# EUROPEAN PATENT APPLICATION

(11) **EP 3 444 344 A1**
(43) Date of publication of application: **20.02.2019**
(21) Application number: 17782459.6
(22) Date of filing: 13.04.2017
(51) Int. Cl.: C12N 15/09, A61K 39/00, A61P 25/28, A61P 37/04, C07K 14/46, C12Q 1/02, G01N 33/15, G01N 33/50, G01N 33/53, A61K 35/12, C12Q 1/37

(54) **AMYLOSPHEROID (ASPD)-LIKE STRUCTURE AND PHARMACEUTICAL COMPOSITION**

(30) Priority: 14.04.2016 JP 2016081030
(71) Applicant: TAO Health Life Pharma Co., Ltd., Kyoto 6068501 (JP)
(72) Inventor: HOSHI, Minako, Kyoto-shi Kyoto 606-8501 (JP); ARAI, Yoshie, Kyoto-shi Kyoto 606-8501 (JP)
(74) Representative: Dehns
(86) International application number: PCT/JP2017/015089
(87) International publication number: WO 2017/179646

(57) **Abstract**

Provided are a cell secreted-type amylospheroids-like structure, a drug and vaccine using the same, as well as a method of producing the same. In one aspect, the present disclosure relates to a production method including a step of culturing, in a culture medium, cells that express an amyloid precursor protein (APP) or a part thereof containing an amyloid beta-protein (Aβ) sequence to obtain a cell secreted-type amylospheroids (ASPD)-like structure in said culture medium.

SEQUENCE LISTING

<110>  TAO Health Life Pharma Co. Ltd.

<120>  Amylospheroids (ASPD)-like Structure and Pharmaceutical
       composition

<130>  H4478-01

<150>  JP 2016-81030
<151>  2016-04-14

<160>  1

<170>  PatentIn version 3.5

<210>  1
<211>  62
<212>  PRT
<213>  Homo sapiens

<400>  1

Ser Glu Val Lys Met Asp Ala Glu Phe Arg His Asp Ser Gly Tyr Glu
1               5                   10                  15

Val His His Gln Lys Leu Val Phe Phe Ala Glu Asp Val Gly Ser Asn
            20                  25                  30

Lys Gly Ala Ile Ile Gly Leu Met Val Gly Gly Val Val Ile Ala Thr
        35                  40                  45

Val Ile Val Ile Thr Leu Val Met Leu Lys Lys Lys Gln Tyr
    50                  55                  60

## Description

### [Technical Field]

The present disclosure relates to an amylospheroids (ASPD)-like structure, a production method thereof and use thereof, a pharmaceutical composition, a production method thereof and use thereof, as well as a screening method.

### [Background Art]

Amylospheroids (ASPD; amylospheroids) each are a spherical Aβ assembly that is formed of about 30 amyloid β proteins (Aβ) aggregated together and has a diameter of approximately 10 nm, and are a structure considered to play an important role in the irreversible stage at which Alzheimer's disease develops.

ASPD were isolated as an in vitro synthesized Aβ assembly (i.e., synthetic ASPD) that exhibited strong neurotoxicity (Non-Patent Document 1). Antibodies specific to this synthetic ASPD have been produced (Patent Documents 1 and 2), and using these antibodies, ASPD formed in vivo (that is, native ASPD) were actually isolated from the brain of a human patient with Alzheimer's disease (Non-Patent Document 1).

Native ASPD and synthetic ASPD likewise selectively induce cell death of mature neurons. It was discovered that the target of ASPD in the neuronal cell death is the synaptic protein "alpha 3 subunit of Na⁺, K⁺-ATPase pump (hereinafter referred to as NAKα3)" that plays a very important role in neuronal survival and function, and it was revealed that the function of NAKa3 decreases due to the binding of ASPD and neurons are excited excessively, which results in death of the neurons (Patent Document 3 and Non-Patent Document 2).

The most correlated with clinical symptoms in Alzheimer's disease is neuronal loss. It was revealed that the amount of native ASPD in the cerebral cortex of an Alzheimer's disease patient with neuronal loss increases relative to the severity of Alzheimer's disease and only a trace amount of native ASPD exists in the cerebellum of an Alzheimer's disease patient with little neuronal loss (Non-Patent Document 3). Therefore, amylospheroids are considered to play an important role in the irreversible stage at which Alzheimer's disease develops. Furthermore, native ASPD have also been detected from the brains of patients with Lewy body dementia (Non-Patent Document 3). Therefore, similarly in Lewy body dementia, ASPD are considered to play an important role in the development thereof.

### [Means for Solving Problem]

Synthetic ASPD, which are considered to be equivalent to native ASPD, can be produced by slowly rotating a liquid containing Aβ (Non-Patent Document 1 and Patent Document 4).

### [Prior Art Documents]

### [Patent Documents]

[Patent Document 1] WO2006/016644
[Patent Document 2] WO2009/057664
[Patent Document 3] WO2013/099806
[Patent Document 4] WO2013/094614

### [Non-Patent Documents]

[Non-Patent Document 1] Hoshi et al., Spherical aggregates of β-amyloid (amylospheroids) show high neurotoxicity and activate tau protein kinase I/glycogen synthase kinase-3β, PNAS May 27, 2003 vol. 100 no. 11 6370-6375
[Non-Patent Document 2] Ohinishi et al., Na, K-ATPase α3 is a death target of Alzheimer patient amyloid-B assembly, PNAS August 11, 2015 vol. 112 no. 32 E4465-E4474
[Non-Patent Document 3] Noguchi et al., Isolation and characterization of patient-derived, toxic, high mass amyloid beta-protein (Abeta) assembly from Alzheimer disease brains, J Biol Chem. 2009 Nov 20;284(47):32895-905

### [SUMMARY OF THE INVENTION]

### [Problems to be Solved by the Invention]

As described above, amylospheroids (ASPD) play an important role in Alzheimer's disease and Lewy body dementia. At present, it is very difficult to purify native ASPD taken from a patient's brain and use it for developing, for example, therapeutic agents. Therefore, if an ASPD-like structure equivalent to or partially equivalent to the native ASPD present in the patient's brain can be easily produced, it is considered to contribute greatly to the research on Alzheimer's disease and Lewy body dementia as well as the development of prophylactic methods and prophylactic agents, and treatment methods and prophylactic/therapeutic agents, with respect to those diseases.

By slowly stirring a liquid containing an amyloid beta-protein (Aβ), a synthetic ASPD that is approximately equivalent to native ASPD can be produced in vitro (Non-Patent Document 1 and Patent Document 4). Synthetic ASPD was found to be able to induce immunity to rabbits without adjuvant, to have a certain vaccine effect in aged monkeys, and to be safe. On the other hand, the synthetic ASPD have also been found to have problems that, for example, the ASPD level fluctuates to some extent for each production lot, the long-term storage of the produced ASPD is difficult, and the scale-up of the production volume is difficult.

Thus, in one aspect, the present disclosure provides an ASPD-like structure and a method of producing the same.

### [Means for solving the Problems]

In one aspect, the present disclosure relates to a method of producing a cell secreted-type ASPD-like structure, including a step of culturing, in a culture medium, cells that express APP or a part thereof containing an Aβ sequence to obtain a cell secreted-type ASPD-like structure in said culture medium.

In another aspect, the present disclosure relates to a cell secreted-type ASPD-like structure that is obtained in a culture medium in which cells have been cultured, with an expression system that expresses APP or a part thereof containing an Aβ sequence having been introduced into the cells, the structure being antigenic to an ASPD-specific antibody.

In another aspect, the present disclosure relates to a pharmaceutical composition including a cell secreted-type ASPD-like structure as an active ingredient.
In another aspect, the present disclosure relates to a vaccine including a cell secreted-type ASPD-like structure.
In another aspect, the present disclosure relates to the use of a cell secreted-type ASPD-like structure as an active vaccine.
In another aspect, the present disclosure relates to the use of a cell secreted-type ASPD-like structure in vaccine production.
In another aspect, the present disclosure relates to the use of a cell secreted-type ASPD-like structure as a reference material in ASPD measurement.

In another aspect, the present disclosure relates to a method of preventing, ameliorating, and/or treating a disease caused by ASPD, the method including administering, to a subject, a cell secreted-type ASPD-like structure, the pharmaceutical composition, or the vaccine.
In another aspect, the present disclosure relates to a method of immunizing a subject against ASPD, a disease caused by ASPD, or Alzheimer's disease and/or Lewy body dementia, the method including administering, to a subject, a cell secreted-type ASPD-like structure, the pharmaceutical composition, or the vaccine.
In another aspect, the present disclosure relates to a method of producing a pharmaceutical composition or a vaccine, the method including combining a cell secreted-type ASPD-like structure with a pharmaceutically acceptable excipient.

In another aspect, the present disclosure relates to a kit containing a cell secreted-type ASPD-like structure and an anti-ASPD antibody.
In another aspect, the present disclosure relates to a method of producing a kit, the method including combining a cell secreted-type ASPD-like structure with an anti-ASPD antibody.
In another aspect, the present disclosure relates to a non-human animal having a cell in which an expression system that expresses APP or a part thereof containing an Aβ sequence has been introduced.

In another aspect, the present disclosure relates to a method of screening a substance that affects the formation of ASPD, the method including using, as an indicator, the formation efficiency of a structure that is formed in a culture medium in which a cell has been cultured, with an expression system that expresses APP or a part thereof containing an Aβ sequence having been introduced into the cell, and the structure being antigenic to an ASPD-specific antibody.
The present disclosure relates to a method of screening a substance that affects the C-terminal cleavage in Aβ, the method using, as an indicator, at least one selected from the group consisting of Aβ40, Aβ41, Aβ42, Aβ43, and combinations thereof, which are secreted in a culture medium in which a cell has been cultured, with an expression system that expresses APP or a part thereof containing an Aβ sequence having been introduced into the cell.

### [Brief Description of Drawings]

[FIG. 1] FIG. 1 is a schematic diagram illustrating expressed APP variants and mutation sites in the mutants thereof.
[FIG. 2] FIG. 2 is a graph showing the results of measuring the ASPD concentrations in the culture media of cells in which APPs of Test Examples 1 to 14 were overexpressed in the expression systems thereof.
[FIG. 3] FIG. 3 is a graph showing the results of measuring the Aβ1-40 concentrations in the culture media of the cells in which APPs of Test Examples 1 to 14 were overexpressed in the expression systems thereof.
[FIG. 4] FIG. 4 is a graph showing the results of measuring the Aβ1-42 concentrations in the culture media of the cells in which APPs of Test Examples 1 to 14 were overexpressed in the expression systems thereof.
[FIG. 5] FIG. 5 is a graph showing the results of measuring the ASPD concentrations in the culture media of cells in which hAPP695sw-G33X was overexpressed.
[FIG. 6] FIG. 6 is a graph showing the results of measuring the Aβ1-40 concentrations in the culture media of cells in which hAPP695sw-G33X was overexpressed.
[FIG. 7] FIG. 7 is a graph showing the results of measuring the Aβ1-42 concentrations in the culture media of cells in which hAPP695sw-G33X was overexpressed.
[FIG. 8] FIG. 8 is a graph showing an example of the results of evaluating immunogenicity of synthetic ASPD.
[FIG. 9] FIG. 9 is a graph showing an example of the results of confirming the neurotoxicity of synthetic ASPD (wild type) and synthetic ASPD (G33L type).
[FIG. 10] FIG. 10 is a graph showing the results of measuring the ASPD concentrations in culture media of cells in which APPs of Test Examples 15 to 36 were overexpressed in the expression systems thereof.
[FIG. 11] FIG. 11 is a graph showing the results of measuring the Aβ1-40 concentrations in the culture media including the cells in which APPs of Test Examples 15 to 36 were overexpressed in the expression systems thereof.
[FIG. 12] FIG. 12 is a graph showing the results of measuring the Aβ1-42 concentrations in the culture media including the cells in which APPs of Test Examples 15 to 36 were overexpressed in the expression systems thereof.

### [Description of Preferred Embodiments]

In one or more embodiments, the present disclosure is based on the confirmation of the presence of an ASPD-like structure in a culture medium in which cells that express an amyloid precursor protein (APP) have been cultured.
In one or more embodiments, the present disclosure relates to providing a cell secreted-type ASPD-like structure that is convenient and can be scaled up.
In one or more embodiments, the cell secreted-type ASPD-like structure according to the present disclosure is excellent in storage stability.
In one or more embodiments, since the cell secreted-type ASPD-like structure according to the present disclosure can be produced with an extremely low content of Aβ monomer, it becomes easy to remove the Aβ monomer by ultrafiltration and thereby it becomes possible to improve the efficiency of producing, for example, a vaccine. Furthermore, in one or more embodiments, since the cell secreted-type ASPD-like structure according to the present disclosure can reduce the content of Aβ monomer, the safety thereof in the case where it is administered to a living body can be improved as compared to, for example, native ASPD or synthetic ASPD.

### [Amylospheroids (ASPD)-Like Structure]

In the present disclosure, the ASPD-like structure refers to a structure with which an ASPD-specific antibody undergoes an antigen-antibody reaction, in other words, a structure that is antigenic to an ASPD-specific antibody.

In the present disclosure, the term amylospheroids (ASPD) simply used by itself may include native ASPD and synthetic ASPD.

Examples of the ASPD-specific antibody include, in one or more embodiments, polyclonal anti-ASPD antibodies and monoclonal anti-ASPD antibodies disclosed in WO2006/016644 and WO2009/057664, and in one or more further embodiments, rabbit polyclonal anti-ASPD antibodies (rpASD1, rpASD2, and rpASD3), mouse monoclonal anti-ASPD antibodies (MASD1, MASD2, and MASD3), and hamster monoclonal anti-ASPD antibodies (haASD1, haASD2, haASD3, haASD4, and haASD5), as well as a humanized monoclonal anti-ASPD antibody (huASD2).

### [Cell Secreted-Type ASPD-Like Structure]

In the present disclosure, a cell secreted-type ASPD-like structure refers to an ASPD-like structure produced by a production method according to the present disclosure. In one or more embodiments, it refers to an ASPD-like structure found in a culture medium of cells in which an expression system of APP or a part thereof containing an Aβ sequence has been introduced. The cell secreted-type ASPD-like structure according to the present disclosure can be considered to be of a cell-secreting type because it is obtained in a culture medium of cells that express APP or a part thereof containing an Aβ sequence.

Therefore, in one aspect, the present disclosure relates to a cell secreted-type ASPD-like structure, the structure being obtained in a culture medium in which cells have been cultured, with an expression system that expresses APP or a part thereof containing an Aβ sequence having been introduced into the cells, and the structure being antigenic to an ASPD-specific antibody.

The cell secreted-type ASPD-like structure according to the present disclosure may be equivalent to ASPD in one or more embodiments and may be partially equivalent to ASPD in one or more other embodiments. The term "partially equivalent" means that it is a structure with which at least an ASPD-specific antibody undergoes an antigen-antibody reaction, in other words, a structure that is antigenic to an ASPD-specific antibody.

The cell secreted-type ASPD-like structure according to the present disclosure is an Aβ assembly in one or more embodiments. In the present disclosure, the term "Aβ" simply used by itself may refer to Aβ40 (Aβ1-40), Aβ41 (Aβ1-41), Aβ42 (Aβ1-42), Aβ43 (Aβ1-43), or all of them or a part thereof.

The cell secreted-type ASPD-like structure according to the present disclosure may have a molecular weight similar to that of ASPD in one or more embodiments and may be 100 kDa or more, or 100 to 700 kDa in one or more other embodiments.

In the cell secreted-type ASPD-like structure according to the present disclosure, in one or more embodiments, the cytotoxicity, i.e., a characteristic that selectively induce cell death in functionally mature neurons, may be equivalent to that of ASPD or may be lower than that of ASPD or may not exist or may be higher than that of ASPD.

In one or more embodiments, the cell secreted-type ASPD-like structure according to the present disclosure may have a shape similar to or a different shape from that of ASPD. In one or more further embodiments, the cell secreted-type ASPD-like structure according to the present disclosure may have a spherical shape with a diameter of 10 to 15 nm in electron microscope observation or may have a shape different from said shape.

### [Method of Producing Cell Secreted-Type ASPD-Like Structure]

In one or more embodiments, the cell secreted-type ASPD-like structure according to the present disclosure can be obtained in a culture medium by culturing cells that express an amyloid precursor protein (APP) or a part thereof containing an amyloid beta-protein (Aβ) sequence in the culture medium.

Therefore, in one aspect, the present disclosure relates to a method of producing a cell secreted-type ASPD-like structure, the method including a step of culturing, in a culture medium, cells that express APP or a part thereof containing an Aβ sequence to obtain a cell secreted-type ASPD-like structure in said culture medium. Hereinafter, the production method described above is also simply referred to as a "production method according to the present disclosure."

In the production method according to the present disclosure, the APP to be expressed in the cells that are cultured may be human APP or APP of a nonhuman animal in one or more embodiments. The human APP may be a splicing variant of any one of hAPP770, hAPP751, and hAPP695. The sequences of APP and Aβ can be obtained from a known database. For example, the accession number of NCBI of hAPP770 is NP_000475 (VERSION NP_000475.1 GI: 4502167). For the sequence of the splicing variant, for example, UniProtKB - P05067 (Modified: November 1,1991 - v3) can be referred to.

In one or more embodiments of the production method according to the present disclosure, in terms of increasing the amount of cell secreted-type ASPD-like structure to be formed, the APP to be expressed in the cells that are cultured is preferably expressed from the expression system of APP introduced into the cells and/or is preferably overexpressed in the cells. In one or more embodiments of the production method according to the present disclosure, the APP to be expressed in the cells that are cultured preferably has a signal sequence of APP in terms of increasing the amount of cell secreted-type ASPD-like structure to be formed.

In one or more embodiments of the production method according to the present disclosure, the APP to be expressed in the cells that are cultured may be of a wild type or may be a mutant APP. Examples of the mutant APP include mutations linked to familial Alzheimer's disease, such as Swedish mutation, Italian mutation, Leuven mutation, Icelandic mutation, London mutation, Iranian mutation, Austrian mutation, German mutation, French mutation, Florida mutation, Iberian mutation, Australian mutation, Belgian mutation, Flemish mutation, Icelandic mutation, British mutation, Tottori mutation, Italian mutation, Arctic mutation, Osaka mutation, Iowa mutation, and Dutch mutation. Among these, it is preferable that a Swedish mutation be included in terms of increasing the amount of cell secreted-type ASPD-like structure to be formed.

Furthermore, in one or more embodiments of the production method according to the present disclosure, in terms of improving the formation efficiency of the cell secreted-type ASPD-like structure, the APP to be expressed in the cells that are cultured has preferably one or more substitution mutations of the glycine of the GXXXG motif in the amino acid sequence from positions 25 to 37 of Aβ. That is, the APP has preferably a substitution mutation of one or more glycines selected from the group consisting of glycines corresponding to positions 25, 29, 33, and 37 of Aβ, and has preferably at least a substitution mutation of a glycine at position 33. The substitution mutations are preferably substitution mutations to leucine, isoleucine, phenylalanine, valine, methionine, tyrosine, or cysteine in terms of improving the formation efficiency of the cell secreted-type ASPD-like structure and in terms of reducing the amount of Aβ in the culture medium. In the present disclosure, "one or more" means 1, 2, 3, or 4, or 1, 2, or 3, or 1 or 2.
In one or more other embodiments, the APP to be expressed in the cells that are cultured has preferably substitution mutations of glycines at positions 33 and 37 of Aβ in terms of improving the formation efficiency of the cell secreted-type ASPD-like structure. In this embodiment, in terms of improving the formation efficiency of the cell secreted-type ASPD-like structure and in terms of reducing the amount of Aβ in the culture medium, the substitution mutations preferably are substitution mutations to leucine, isoleucine, phenylalanine, valine, methionine, tyrosine, or cysteine, and more preferably at least one of positions 33 and 37 is a substitution mutation to isoleucine.

In one or more embodiments of the production method according to the present disclosure, in terms of improving the formation efficiency of the cell secreted-type ASPD-like structure, examples of the APP to be expressed in the cells that are cultured include those having a Swedish mutation and a substitution mutation of one or more glycines selected from the group consisting of glycines corresponding to positions 25, 29, 33, and 37 of Aβ. In one or more other embodiments, examples of the APP in the present disclosure include those having, in hAPP770 or hAPP695, a Swedish mutation and a substitution mutation of one or more glycines selected from the group consisting of glycines corresponding to positions 25, 29, 33, and 37 of Aβ. In one or more still other embodiments, examples of the APP in the present disclosure include those having, in hAPP770 or hAPP695, a Swedish mutation and a substitution mutation of glycine corresponding to position 33 of Aβ. In one or more yet other embodiments, examples of the APP in the present disclosure include those having, in hAPP770 or hAPP695, a Swedish mutation and substitution mutations (preferably at least one of them is a substitution mutation to isoleucine) of glycines corresponding to positions 33 and 37 of Aβ. In one or more embodiments of the production method according to the present disclosure, the APP to be expressed in the cells that are cultured may have other mutations as long as they do not significantly hinder the formation of the ASPD-like structure. Examples of other mutations may include mutations to, for example, modified amino acids, unnatural amino acids, and D-amino acids. In one or more embodiments, examples of the modified amino acids include amino group modification, carboxyl group modification, thiol group modification, hydroxyl group modification, glycation modification, and PEGylation modification.

In one or more embodiments of the production method according to the present disclosure, the APP to be expressed in the cells that are cultured may be a partial peptide that is a part of APP and contains an amyloid beta-protein (Aβ) sequence (hereinafter, said partial peptide is also simply referred to as a "part of APP"). This is because ASPD are a form of the assembly of Aβ cut out from APP.
In one or more embodiments of the production method according to the present disclosure, the part of APP to be expressed in the cells that are cultured may be in an N-terminal cleaved form and in one or more further embodiments, may be in a form in which the N terminus is cleaved at the cleavage site of β-secretase.
In one or more embodiments of the production method according to the present disclosure, the part of APP to be expressed in the cells that are cultured is, for example, a portion including positions 1 to 43, 1 to 42, or 1 to 40 of Aβ and in one or more further embodiments, it has, for example, a portion including positions 1 to 43, 1 to 42, or 1 to 40 of Aβ and the signal sequence of the APP.

APP or a part thereof in cells that are cultured can be expressed in the cells in which an expression system capable of expressing APP or a part thereof has been introduced. APP or a part thereof may be expressed by a transient expression system or by a stably expressing cell line. In terms of improving the amount of the ASPD-like structure to be formed, the expression of APP or a part thereof is preferably high expression or overexpression as long as it does not hinder the formation of the ASPD-like structure. The expression system of the APP or a part thereof may be controllable.

In one or more embodiments, the expression system capable of expressing APP or a part thereof is, for example, an expression cassette including a nucleic acid in which an expression regulatory sequence appropriate for the host cell to be introduced is operatively linked to a sequence that encodes the APP or a part thereof. Examples of the expression regulatory sequence include promoters, enhancers, and transcription terminators, and in addition, include start codons, intron splicing signals, and stop codons.

In one or more embodiments, in the expression system of APP or a part thereof, an expression vector appropriate for the cells (host) to be expressed can be suitably selected and introduced thereinto. In one or more embodiments, examples of said expression vector include a vector having the expression cassette described above. In one or more embodiments, examples of the expression vector include plasmids, cosmids, YACS, virus (for example, adenovirus, retrovirus, and episomal EBV) vectors, and phage vectors.

The cells to be cultured in the production method according to the present disclosure are cells that express APP or a part thereof. In terms of improving the amount of the cell secreted-type ASPD-like structure to be formed, the cells to be cultured in the production method according to the present disclosure are preferably cells in which an expression system capable of expressing APP or a part thereof has been introduced, and more preferably cells that highly express or overexpress or can highly express or overexpress APP or a part thereof. In another embodiment of the production method according to the present disclosure, the cells that express APP or a part thereof may be cells in which genomic APP genes are overexpressed by gene transfer or genome editing.

In one or more embodiments, the cells to be cultured in the production method according to the present disclosure are preferably cells capable of producing Aβ, more preferably cells in which both γ-secretase and β-secretase are expressed.

In one or more embodiments, examples of the host cell into which the expression system of APP or a part thereof is introduced include animal cells, plant cells, insect cells, microorganisms, and cell lines thereof. Examples of animal cells include mammalian cells, human cells, and non-human mammalian cells.
In one or more embodiments, the host cell into which the expression system of APP or a part thereof is introduced is preferably a cell line in terms of handleability. Examples of specific cell lines include, but not limited to, CHO cells, HEK293 cells, and Neuro2a cells.

With respect to the conditions for culturing cells that express APP or a part thereof in the production method according to the present disclosure, those skilled in the art can suitably determine, for example, the medium, temperature, and CO₂ concentration according to the type of said cells, the type of the expression system introduced into said cells, and the form of introduction of the expression system (transient introduction or stable introduction) so that the APP or a part thereof incorporated into the expression system is expressed (preferably overexpressed).

In one or more embodiments, an example of the culture using a transient APP expression system includes culturing and transducing in a growth medium, then replacing it with a serum-free medium, and culturing it for 24 hours to 48 hours. Then, after the culturing as described above, a cell secreted-type ASPD-like structure is obtained in the medium.

When the cells are animal cells, examples of the medium include Medium 199 medium, Eagle's Minimum Essential Medium (EMEM), αMEM, Dulbecco's modified Eagle's Medium (DMEM), Ham's F12 medium, RPMI 1640 Medium, Fischer's medium, and mixed media thereof. These media may contain serum or serum substitutes or may be serum-free. The medium may also contain one or more substances such as lipids, amino acids, non-essential amino acids, vitamins, growth factors, low molecular weight compounds, antibiotics, antioxidants, pyruvic acid, buffers, and inorganic salts as required.

In one or more embodiments, the production method according to the present disclosure further includes collecting the cell secreted-type ASPD-like structure from the culture medium.
In one or more embodiments, the cell secreted-type ASPD-like structure in the culture medium can be collected by a step of collecting a holding solution obtained by ultrafiltration of 50 kDa or 100 kDa of a filtrate obtained using a filter with a pore size of 0.22 µm. However, the method of collecting ASPD is not limited to this method.

The production method according to the present disclosure makes it easy to produce a cell secreted-type ASPD-like structure and thereby, in one or more embodiments, makes it easy to develop an active vaccine therapy using the cell secreted-type ASPD-like structure itself as an antigen, pharmaceutical compositions that function as active vaccines, and neuronal cell death inhibitors.

### [Vaccine]

It was reported that as a result of an attempt to introduce Aβ itself, which constitutes ASPD, into a living body to impart immunity, a part (residues at positions 16 to 33) of the sequence of Aβ activates T cells and causes an inflammatory reaction (Monsonego, A. et al. J. Clin. Invest. 112:415-422 (2003), Monsonego, A. et al. PNAS 103:5048-5053 (2006)). Therefore, it is difficult to use Aβ itself as a vaccine.

On the other hand, data has been obtained that in ASPD, the sequence (residues at positions 16 to 33) of Aβ which activates T cells is folded inside a spherical structure and is almost concealed (Ohnishi et al. PNAS 112:E4465-E4474 (2015)) and therefore, ASPD do not cause inflammatory T cell responses that are problematic with Aβ vaccines.

With respect to synthetic ASPD, it was found that rabbits were immunized without an adjuvant and an immunizing dose thereof as small as 1/5 to 1/10 of normal peptide antigen was sufficient. Furthermore, it was also found that when an old cynomolgus monkey was immunized by subcutaneous administration using ASPD, the brain glucose metabolism (neural activity) thereof increased.

Based on these findings, it can be said that ASPD can be used as a highly safe active vaccine. The cell secreted-type ASPD-like structure according to the present disclosure can also be used as an active vaccine and can be used for the production of vaccines.

Therefore, in one aspect, the present disclosure relates to a pharmaceutical composition containing ASPD as an active ingredient or to a vaccine containing ASPD.

When trying to produce a pharmaceutical composition, such as a vaccine, using synthetic ASPD, the following problems were found. That is, in the conventional method of producing synthetic ASPD, the amount of ASPD fluctuated to some extent for each production lot, the long-term storage of the ASPD produced was difficult, the production amount was difficult to be scaled up, and further it was necessary to remove residual Aβ after producing the synthetic ASPD, which resulted in a decrease in production efficiency.
In one or more embodiments, a pharmaceutical composition such as a vaccine produced using the cell secreted-type ASPD-like structure according to the present disclosure can solve one or a part, or more of these problems.

Accordingly, in one aspect, the present disclosure relates to a pharmaceutical composition containing a cell secreted-type ASPD-like structure as an active ingredient. One or more embodiments of the pharmaceutical composition according to the present disclosure include a vaccine containing a cell secreted-type ASPD-like structure. The pharmaceutical composition and vaccine according to the present disclosure may contain pharmaceutically acceptable excipients and/or adjuvants.

In one or more embodiments, the pharmaceutical composition and vaccine according to the present disclosure may be used for the prevention, amelioration, and/or treatment of diseases caused by ASPD. In one or more embodiments, the pharmaceutical composition and vaccine according to the present disclosure may be used for the prevention, amelioration, and/or treatment of Alzheimer's disease and/or Lewy body dementia.
In one or more embodiments, the pharmaceutical composition or vaccine according to the present disclosure may be administered to a subject who is likely to suffer from a disease caused by ASPD, a subject who may have developed it, or a subject who is suffering from it. In one or more embodiments, the pharmaceutical composition according to the present disclosure may be administered to a subject who is likely to suffer from Alzheimer's disease and/or Lewy body dementia, a subject who may have developed it, or a subject who is suffering from it. Such subjects include mammals, humans, and non-human mammals.

One or more embodiments will be described in which a pharmaceutical composition or vaccine according to the present disclosure allows subjects to acquire immunity against ASPD, diseases caused by ASPD, or Alzheimer's disease and/or Lewy body dementia. In one or more embodiments, examples of the method of administering the pharmaceutical composition or vaccine according to the present disclosure may include intramuscular, intraperitoneal, intradermal, or subcutaneous injection, or transmucosal administration through the oral tract, gastrointestinal tract, respiratory tract, or genitourinary tract. Examples of the dose of the cell secreted-type ASPD-like structure in the pharmaceutical composition or vaccine according to the present disclosure include amounts that induce an immunoprotective response without causing a significant side effect to the subject to be administered. In one or more embodiments, the dose of the pharmaceutical composition or vaccine according to the present disclosure is estimated to be a dose that contains the cell secreted-type ASPD-like structure in the range of 0.01 µg to 10 mg, 0.1 to 1000 µg, 1 to 100 µg, 5 to 50 µg, or 5 to 25 µg. Following the initial administration, one or several booster doses may be administered at sufficient intervals.

Accordingly, in one aspect, the present disclosure relates to a method of preventing, ameliorating, and/or treating a disease caused by ASPD (for example, Alzheimer's disease and/or Lewy body dementia), the method including administering, to a subject, a cell secreted-type ASPD-like structure or a pharmaceutical composition or vaccine according to the present disclosure.
In another aspect, the present disclosure relates to a method of immunizing a subject against ASPD, diseases caused by ASPD, or Alzheimer's disease and/or Lewy body dementia, the method including administering, to a subject, a cell secreted-type ASPD-like structure or a pharmaceutical composition or vaccine according to the present disclosure.

Furthermore, in one aspect, the present disclosure relates to a method of producing a pharmaceutical composition or a vaccine, the method including combining a cell secreted-type ASPD-like structure with an excipient and/or an adjuvant.
In another aspect, the present disclosure relates to a method of producing a pharmaceutical composition containing a cell secreted-type ASPD-like structure as an active ingredient or a vaccine having a cell secreted-type ASPD-like structure, the method including a step of producing a cell secreted-type ASPD-like structure by a production method according to the present disclosure.

In another aspect, the present disclosure relates to a kit for use in a production method according to the present disclosure, the kit including a cell line in which an expression system of APP or a part thereof has been introduced. The kit according to the present disclosure facilitates the production of the cell secreted-type ASPD-like structure. The kit according to the present disclosure may further include at least one of a culture vessel, a medium, and instructions. In one or more embodiments, the kit for use in a production method according to the present disclosure may include a vector having an expression system of APP or a part thereof in place of a cell line in which an expression system of APP or a part thereof has been introduced.

### [Model Animals]

In another aspect, the present disclosure relates to a non-human animal having a cell in which an expression system that expresses APP or a part thereof containing an Aβ sequence has been introduced. Since the cell secretes a cell secreted-type ASPD-like structure, the non-human animal can be a model animal for a disease caused by ASPD, for example, Alzheimer's disease and/or Lewy body dementia. In terms of obtaining said model animal, the cell is preferably a brain cell or a neuron. The method of introducing the expression system is not particularly limited, but examples thereof include a method using a gene transfer technique and a method using a genome editing technique. Examples of non-human animals include non-human mammals and non-human primates. One or more embodiments of this aspect include a genetically modified animal having cells genetically modified to express APP or a part thereof containing an Aβ sequence.

In the model animal of this aspect, the APP that is expressed in a genetically modified cell may be, for example, a mutant of APP or a mutant of Aβ as disclosed in the present disclosure. That is, the APP that is expressed in the cell, as described above, has preferably a substitution mutation of one or more glycines selected from the group consisting of glycines corresponding to positions 25, 29, 33, and 37 of Aβ, and has preferably a substitution mutation of glycine at least at position 33. The substitution mutation is preferably a substitution mutation to leucine, isoleucine, phenylalanine, valine, methionine, tyrosine, or cysteine.

In one or more other embodiments, it is preferable that the APP that is expressed in the cell have substitution mutations of glycines at positions 33 and 37 of Aβ. In the present embodiment, it is preferable that the substitution mutations be substitution mutations to leucine, isoleucine, phenylalanine, valine, methionine, tyrosine, or cysteine, and it is more preferable that the substitution mutation at least at one of positions 33 and 37 be a substitution mutation to isoleucine.

### [Reference Material]

In one or more embodiments, the cell secreted-type ASPD-like structure can be used as a reference material of ASPD in ASPD measurement. In one or more other embodiments, the cell secreted-type ASPD-like structure can be used in a kit for measuring ASPD. That is, in one aspect, the present disclosure relates to a kit including a cell secreted-type ASPD-like structure and an anti-ASPD antibody. Examples of the anti-ASPD antibody include the above-mentioned ASPD-specific antibody. Furthermore, in one aspect, the present disclosure relates to a method of producing a kit, the method including combining a cell secreted-type ASPD-like structure with an anti-ASPD antibody. Such a kit can be used, for example, for ASPD measurement.

### [Screening Method 1]

In one aspect, the present disclosure relates to a method of screening a substance that affects the formation of ASPD, the method including using, as an indicator, the formation efficiency of a structure (that is, a cell secreted-type ASPD-like structure) that is antigenic to an ASPD-specific antibody, the structure being formed in a culture medium in which cells have been cultured, with an expression system that expresses APP or a part thereof containing an Aβ sequence having been introduced into the cells. In one or more embodiments, the indicator described above may be the amount of the cell secreted-type ASPD-like structure to be formed or may be an amount relative to Aβ40, Aβ41, Aβ42, Aβ43 or a total amount thereof. The screening method of this embodiment may include comparing the formation efficiency of the cell secreted-type ASPD-like structure obtained in the case of adding a test substance to a culture medium with that obtained in the case where it was not added, to judge whether or not the substance affects the formation of ASPD. For example, if said formation efficiency decreases when a test substance was added to a culture medium, it can be judged that said substance has an effect of inhibiting the formation of ASPD. Furthermore, for example, if the formation efficiency increases when a test substance was added to a culture medium, it can be judged that said substance has an effect of promoting the formation of ASPD.

In the screening method of this aspect, the APP to be expressed in the cells that are cultured may be, for example, a mutant of APP or a mutant of Aβ as disclosed in the present disclosure.

### [Screening Method 2]

In one aspect, the present disclosure relates to a method of screening a substance that affects the C-terminal cleavage in Aβ, the method including using, as an indicator, at least one Aβ selected from the group consisting of Aβ40, Aβ41, Aβ42, Aβ43 and combinations thereof, which are formed in a culture medium in which cells have been cultured, with an expression system that expresses APP or a part thereof containing an Aβ sequence having been introduced into the cells.

In one or more embodiments, the indicator may be the secretion amount of Aβ40, Aβ41, Aβ42, Aβ43, or combinations thereof or the concentration thereof in a culture medium, or may be, for example, a relative ratio such as a ratio of Aβ42 to Aβ40 (Aβ42/Aβ40). The screening method of this embodiment may include comparing the indicator obtained in the case of adding a test substance to a culture medium with that obtained in the case where it was not added, to judge whether or not the substance affects the C-terminal cleavage of Aβ or whether or not the substance affects γ secretase. For example, if "Aβ42/Aβ40" decreases when the test substance was added to a culture medium, it can be judged that said substance has an effect of inhibiting the production of Aβ42.

In the screening method of this aspect, the APP to be expressed in the cells that are cultured may be, for example, a mutant of APP or a mutant of Aβ as disclosed in the present disclosure.

### [Mutant Synthetic ASPD]

In still another aspect, the present disclosure relates to a mutant synthetic ASPD. In the present disclosure, the mutant synthetic ASPD refers to a synthetic ASPD-like structure that is synthesized using a mutant Aβ by a production method similar to the method of producing a synthetic ASPD. That is, in the present disclosure, the mutant synthetic ASPD refers to a synthetic ASPD-like structure that is formed by stirring a liquid containing a mutant Aβ having one or more substitution mutations of glycines of the GXXXG motif in the amino acid sequence of positions 25 to 37 of Aβ. In terms of improving the formation efficiency of the mutant synthetic ASPD, the above-mentioned liquid may contain, for example, a plasticizer such as a phthalate ester. In one or more embodiments, the mutant synthetic ASPD can be synthesized by a production method including dissolving the mutant Aβ in an organic solvent containing the plasticizer, diluting the solution of the mutant Aβ with an aqueous solution, and stirring the solution thus diluted.

The mutant Aβ has preferably one or more substitution mutations of glycines selected from the group consisting of glycines corresponding to positions 25, 29, 33, and 37 of Aβ and has preferably a substitution mutation of at least a glycine at position 33. In terms of improving the formation efficiency of the synthetic ASPD, preferred substitution mutations include substitution mutations to leucine, isoleucine, phenylalanine, valine, methionine, tyrosine, and cysteine.

In one or more other embodiments, the mutant Aβ preferably has substitution mutations of glycines at positions 33 and 37 of Aβ. In the present embodiment, in terms of improving the formation efficiency of the synthetic ASPD, the substitution mutations are preferably substitution mutations to leucine, isoleucine, phenylalanine, valine, methionine, tyrosine, or cysteine, and more preferably a substitution mutation to isoleucine at least at one of positions 33 and 37.

In one or more embodiments, the mutant ASPD according to the present disclosure is antigenic to an ASPD-specific antibody. Furthermore, in one or more other embodiments, the mutant ASPD according to the present disclosure is cytotoxic to mature neurons.

In one or more embodiments, the present disclosure may relate to the following:
[1] A method of producing a cell secreted-type amylospheroids (ASPD)-like structure, the method including a step of culturing, in a culture medium, cells that express an amyloid precursor protein (APP) or a part thereof containing an amyloid beta-protein (Aβ) sequence to obtain a cell secreted-type ASPD-like structure in said culture medium.
[2] The production method according to [1], further including collecting the cell secreted-type ASPD-like structure from the culture medium.
[3] The production method according to [1] or [2], wherein the cells are cells in which an expression system of APP or a part thereof containing an Aβ sequence has been introduced.
[4] The production method according to any one of [1] to [3], wherein the APP is of a wild-type or is a mutant of the wild-type having one or more amino acid mutations.
[5] The production method according to any one of [1] to [4], wherein the APP is a wild-type or a mutant APP having an amino acid mutation in glycine in the GXXXG motif of the Aβ amino acid sequence.
[6] A cell secreted-type ASPD-like structure obtained in a culture medium in which cells have been cultured, with an expression system that expresses APP or a part thereof containing an Aβ sequence having been introduced into the cells, the structure being antigenic to an ASPD-specific antibody.
[7] A pharmaceutical composition including, as an active ingredient, a cell secreted-type ASPD-like structure according to [6].
[8] A vaccine including a cell secreted-type ASPD-like structure according to [6].
[9] A kit including a cell secreted-type ASPD-like structure according to [6] and an anti-ASPD antibody.
[10] Use of a cell secreted-type ASPD-like structure according to [6] as an active vaccine.
[11] Use of a cell secreted-type ASPD-like structure according to [6] in vaccine production.
[12] Use of a cell secreted-type ASPD-like structure according to [6] as a reference material in ASPD measurement.
[13] A method of preventing, ameliorating, and/or treating a disease caused by ASPD, the method including administering, to a subject, a cell secreted-type ASPD-like structure according to [6], a pharmaceutical composition according to [7], or a vaccine according to [8].
[14] The method according to [13], wherein the disease caused by ASPD is Alzheimer's disease and/or Lewy body dementia.
[15] A method of immunizing a subject against ASPD, a disease caused by ASPD, or Alzheimer's disease and/or Lewy body dementia, the method including administering, to the subject, a cell secreted-type ASPD-like structure according to [6], a pharmaceutical composition according to [7], or a vaccine according to [8].
[16] A method of producing a pharmaceutical composition or a vaccine, the method including combining a cell secreted-type ASPD-like structure according to [6] with a pharmaceutically acceptable excipient.
[17] A method of producing a kit, the method including combining a cell secreted-type ASPD-like structure according to [6] with an anti-ASPD antibody.
[18] A non-human animal, having a cell in which an expression system that expresses APP or a part thereof containing an Aβ sequence has been introduced.
[19] A method of screening a substance that affects the formation of ASPD, the method including using, as an indicator, the formation efficiency of a structure that is formed in a culture medium in which cells have been cultured, with an expression system that expresses APP or a part thereof containing an Aβ sequence having been introduced into the cells, and the structure being antigenic to an ASPD-specific antibody.
[20] A method of screening a substance that affects the C-terminal cleavage in Aβ, the method including using, as an indicator, at least one selected from the group consisting of Aβ40, Aβ41, Aβ42, Aβ43, and combinations thereof, which are secreted in a culture medium in which cells have been cultured, with an expression system that expresses APP or a part thereof containing an Aβ sequence having been introduced into the cell.
[21] A synthetic ASPD-like structure formed by stirring a liquid containing a mutant Aβ having one or more substitution mutations of the glycines of the GXXXG motif in the amino acid sequence of positions 25 to 37 of Aβ.

Hereinafter, one or more embodiments of the present invention are further described using examples.

### [Examples]

### 1. Construction of cultured cells that express hAPP

### (1-1) Human amyloid precursor protein (hAPP)

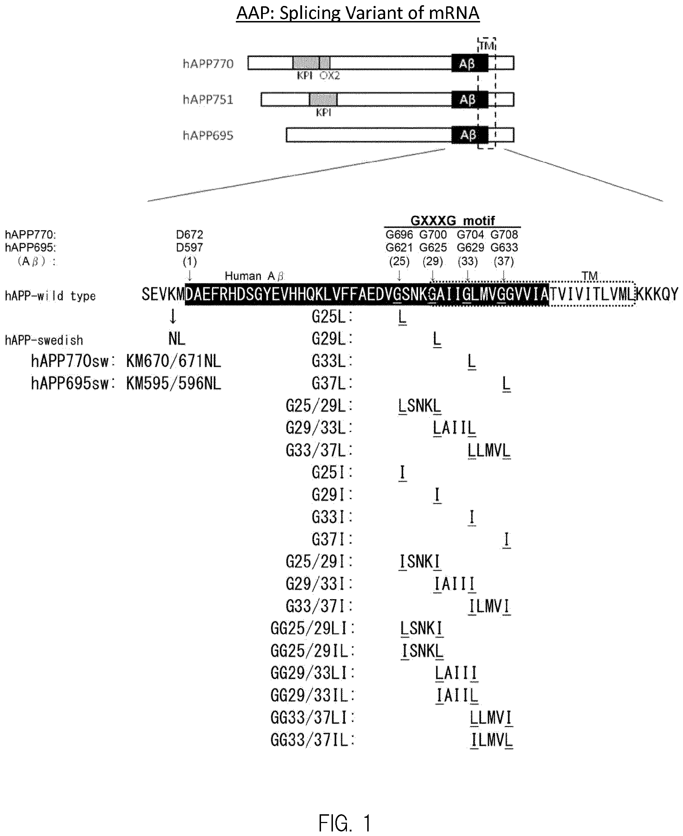

As the amyloid precursor protein (APP) to be expressed, APPs of Test Examples 1 to 14 shown in Table 1 below and FIG. 1 were used. The sequence (SEQ ID NO: 1) of FIG. 1 is a partial amino acid sequence of hAPP containing human Aβ.

The APPs of Test Examples 1 to 7 are hAPP770 wild type, hAPP770 Swedish mutant type, and hAPP770 Swedish type in which 1 or 2 glycines of the GXXXG motif were mutated.

The APPs of Test Examples 8 to 14 are hAPP695 wild type, hAPP695 Swedish mutant type, and hAPP695 Swedish type in which 1 or 2 glycines of the GXXXG motif were mutated.

**[Table 1]**

| Table 1 | APP Expressed | Mutations in APP | |
|---|---|---|---|
| Test Example 1 | hAPP770-wild type | - | |
| Test Example 2 | hAPP770-swedish (SW) | KM670/671NL | |
| Test Example 3 | hAPP770-sw-G25/29L | KM670/671NL | G696/700L |
| Test Example 4 | hAPP770-sw-G29/33L | KM670/671NL | G700/704L |
| Test Example 5 | hAPP770-sw-G33/37L | KM670/671NL | G704/708L |
| Test Example 6 | hAPP770-sw-G33L | KM670/671NL | G704L |
| Test Example 7 | hAPP770-sw-G33I | KM670/671NL | G704I |
| Test Example 8 | hAPP695-wild type | - | |
| Test Example 9 | hAPP695-swedish (SW) | KM595/596NL | |
| Test Example 10 | hAPP695-sw-G25/29L | KM595/596NL | G621/625L |
| Test Example 11 | hAPP695-sw-G29/33L | KM595/596NL | G625/629L |
| Test Example 12 | hAPP695-sw-G33/37L | KM595/596NL | G629/633L |
| Test Example 13 | hAPP695-sw-G33L | KM595/596NL | G629L |
| Test Example 14 | hAPP695-sw-G33I | KM595/596NL | G629I |

### (1-2) Cultured cells that overexpress hAPP

CHO cells that overexpress hAPP in Test Examples 1 to 14 described above were prepared as follows.

CHO/K1 cells were seeded in a 12-well plate at a density of 2.5 × 10⁴ cells/cm² in a growth medium and then were maintained at 37°C in 5% CO₂. After 24 hours, an expression vector was introduced into the CHO/K1 cells using a gene transfer reagent (FuGENE HD). The introduction method included mixing 25 µL of Opti-MEM and 0.5 µg of plasmid DNA together per well, further adding 1.5 µL of FuGENE HD, and then reacting them at room temperature for ten minutes. During the reaction, 0.5 mL of the growth medium was replaced, and after completion of the reaction, 25 µL of the reaction solution was added thereto. After 24 hours, the growth medium was replaced with 1 mL of new growth medium and the cells were further cultured. Growth medium: Ham's F-12 medium containing 10% FBS supplemented with 100 units/mL of penicillin and 100 µg/mL of streptomycin.

### 2. Formation and Collection of ASPD

### (2-1) Culture of cultured cells that overexpress hAPP and collection of supernatant

Forty eight hours after the gene transfer, the cells were washed twice with 1 × PBS(-) and then the culture medium was replaced with 1 mL of serum-free medium. Twenty four hours after the replacement with the serum-free medium, the culture supernatant filtered through a 0.22-µm filter was collected, immediately frozen in liquid nitrogen, and then stored at -80°C.
Serum-free medium: DMEM/F-12 with 1 × ITS-X added thereto

### (2-2) Analysis

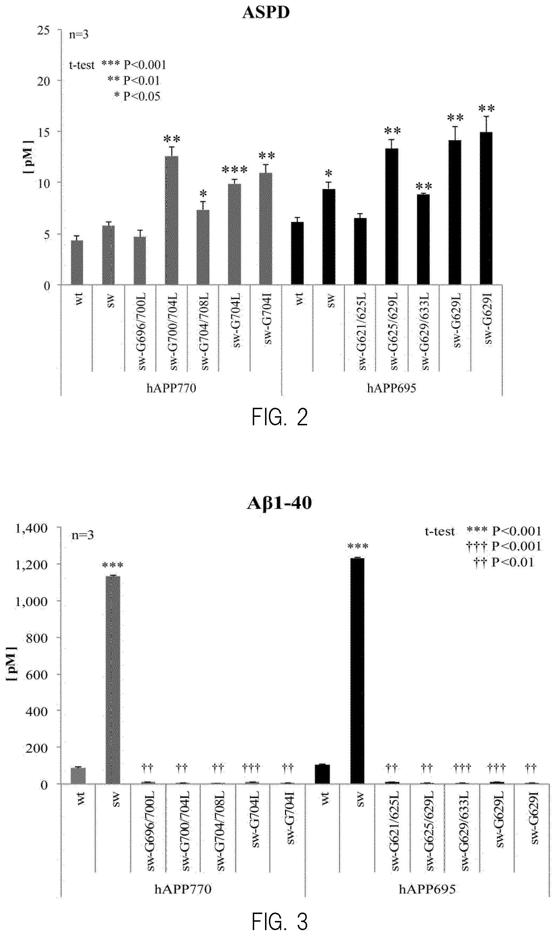

The culture medium in which the APP overexpressing cells had been cultured was collected and the contents of ASPD, Aβ1-40, and Aβ1-42 in the culture medium were confirmed by the ELISA method under the following conditions. The results are shown in FIGS. 2 to 4.

### [ASPD ELISA]

The collected culture supernatant was quantified by a sandwich ELISA using two types of anti-ASPD antibodies (rpASD1 and MASD3).

Specifically, on the day before the measurement, anti-ASPD polyclonal antibody (rpASD1) was dispensed into a white 96-well plate to be 500 ng/well and then was immobilized overnight at 4°C. The next day, after being washed three times with PBS-T, the wells were blocked for 30 minutes with 3% BSA and then were washed again three times. 100 µL of standard solution or sample was dispensed into each well, incubated at room temperature for one hour, and washed three times. Then an anti-ASPD monoclonal antibody (MASD3) was added thereto to be 100 ng/well, and this was further incubated at room temperature for one hour. After this was washed again three times, a secondary antibody (anti-mouse IgG-HRP) diluted 1/10,000 was dispensed at 100 µL/well and then was incubated at room temperature for one hour. After this was washed three times, 100 µL of a luminescent substrate was added thereto, which was reacted for one minute with shielded light, and then luminescence was detected with a luminometer.

### [Aβ ELISA]

The collected culture supernatant was subjected to measurements using commercially available Aβ monomer ELISA kits. The kits used herein are as described below. The measurements were performed according to the instructions attached to the kits.
Aβ1-40: Human β Amyloid (1-40) ELISA Kit Wako (Wako # 292-62301)
Aβ1-42: Human β Amyloid (1-42) ELISA Kit Wako High Sensitivity Product (Wako # 296-64401)

As shown in FIGS. 2 to 4, formation of ASPD was observed in the culture medium in all the cases where the APPs of Test Examples 1 to 14 were overexpressed.

When hAPP770 and hAPP695 were compared to each other, there was no significant difference caused by the mutation, but hAPP695 tended to have a slightly higher ASPD concentration in the culture supernatant. With respect to the mutation sites and the number of mutations, G/L mutation at G704 or G629 was considered to be important for ASPD formation regardless of whether the mutation occurred at two sites or one site. Furthermore, compared to the wild type (wt), the Swedish (sw) mutation secreted more amyloid, but it was suggested that when a mutation was further introduced into the GXXXG motif, most amyloids were used as a component of ASPD.

### 3. Overexpression of hAPP695sw-G33X

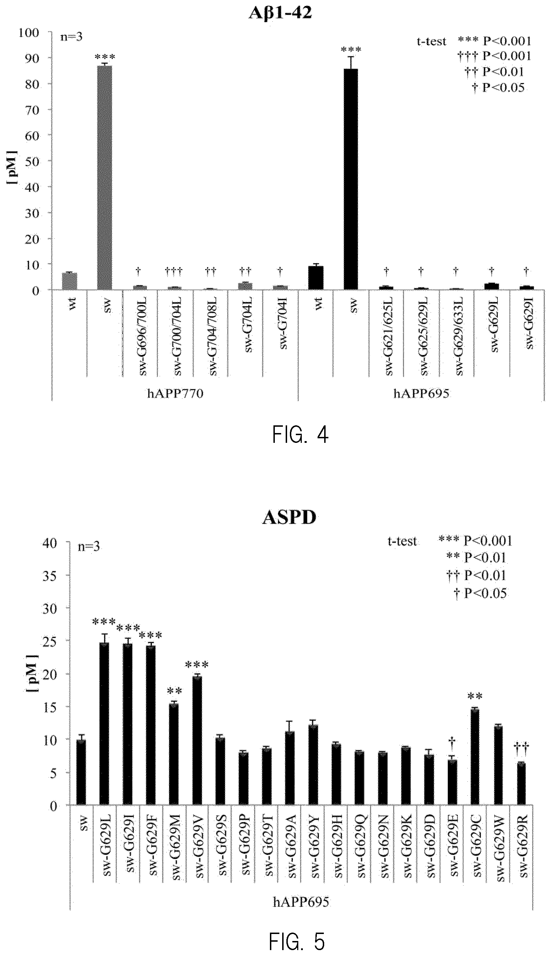

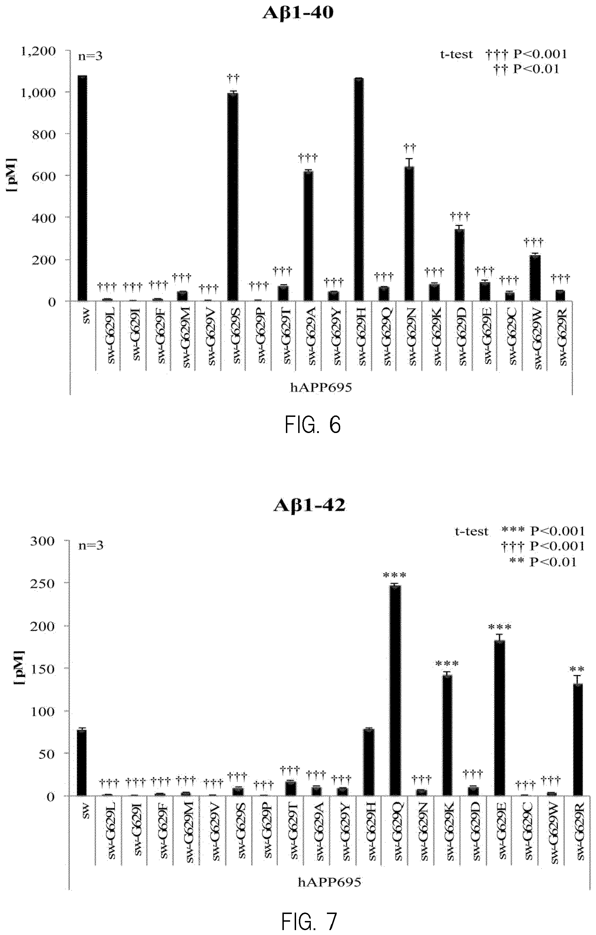

Cells that overexpress APP in which glycine at position 629 (position 33 of Aβ) of hAPP695 Swedish mutant type was mutated to 19 types of amino acids were prepared in the same manner as in section (1-2) above, and they were cultured in the same manner as in section 2 above. Then, the contents of ASPD, Aβ1-40, and Aβ1-42 in the culture medium were confirmed. The results are shown in Table 2 and FIGS. 5 to 7.

**[Table 2]**

| APP | ASPD-Like Structure |
|---|---|
| | Ratio to sw (%) |
| sw | 100 |
| sw-G629L | 251 |
| sw-G629I | 249 |
| sw-G629F | 245 |
| sw-G629M | 156 |
| sw-G629V | 196 |
| sw-G629S | 103 |
| sw-G629P | 81 |
| sw-G629T | 87 |
| sw-G629A | 114 |
| sw-G629Y | 123 |
| sw-G629H | 94 |
| sw-G629Q | 82 |
| sw-G629N | 81 |
| sw-G629K | 89 |
| sw-G629D | 78 |
| sw-G629E | 70 |
| sw-G629C | 147 |
| sw-G629W | 121 |
| sw-G629R | 65 |

As shown in Table 2 and FIGS. 5 to 7, it was confirmed that when the glycine at position 629 (position 33 of Aβ) in the hAPP695 Swedish mutant type was substituted with a predetermined amino acid such as leucine, isoleucine, phenylalanine, methionine, valine, tyrosine, or cysteine, the amount of Aβ in the culture medium was remarkably reduced and the amount of the cell secreted-type ASPD-like structure formed therein was able to be increased. On the other hand, it was confirmed that when the glycine was substituted with proline (or threonine), the concentrations of Aβ1-40, Aβ1-42, and ASPD-like structure in the culture medium all decreased. From these results, it was suggested that depending on the mutation at position 629 (position 33 of Aβ) to these amino acids, excision of Aβ from APP was inhibited and the formation of ASPD-like structure was suppressed. Thus, it was considered to be also possible to use mutants for inhibitor screening with respect to both amyloid β production and ASPD formation.

### 3. Production of cell secreted-type ASPD-like structure using other host cells

In addition to CHO, similarly in the case of cell lines of HEK293 and Neuro2a, it was observed that when cells in which an expression system of APP or a part thereof containing an Aβ sequence had been introduced were used, an ASPD-like structure was formed in the cells.

### 4. Confirmation of safety of cell secreted-type ASPD-like structure: Storage stability at -80°C

A cell secreted-type ASPD structure obtained by expressing hAPP695-sw-G33I of Test Example 14 was stored at -80°C for 0 month, 3 months, and 6 months. The samples thus obtained were subjected to measurement performed with a sandwich ELISA using two types of ASPD-specific antibodies (neutralizing antibodies) to prepare a calibration curve.

As a result, the slope of the primary regression line of the calibration curve showed approximately the same value with respect to the three samples. Therefore, the reactivity to the two types of neutralizing antibodies used in the sandwich ELISA was stable and thus it was considered that there was no deterioration due to storage.

On the other hand, after being stored at -80°C, the synthetic ASPD produced by a conventional method had a large variation in the slope of the primary regression line and also a large variation in the magnitude of the signal with respect to the ASPD concentration. Thus, the synthetic ASPD are considered to have low storage stability.

### 5. Immunogenicity of synthetic ASPD

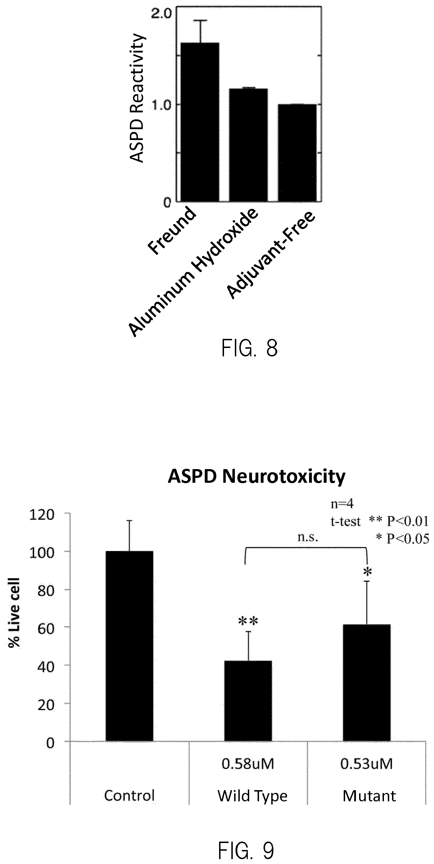

New Zealand white rabbits (3 per each condition) were immunized six times with 100 µg of synthetic ASPD using Freund's adjuvant, aluminum hydroxide adjuvant, and no adjuvant. Thereafter, the whole blood was collected and the reactivity to the synthetic ASPD in serum was evaluated by dot blot. An example of the results is shown in FIG. 8. It was found that sufficient reactivity was obtained in all the rabbits even without using adjuvant.

### 6. Neurotoxicity of synthetic ASPD and mutant synthetic ASPD (G33L)

The neurotoxicity of synthetic ASPD produced using a wild type Aβ1-42 was compared with the neurotoxicity of mutant synthetic ASPD (G33L) produced using G33L mutant Aβ1-42.

Specifically, a fixed concentration of synthetic ASPD (0.58 µM) and mutant synthetic ASPD (0.53 µM) each were administered to adult rat hippocampus-derived primary cultured neurons. After being left alone overnight, a fluorescent reagent (CyQUANT manufactured by Thermo Fisher) was added thereto, and then the cell viability was confirmed using a confocal image cytometer CQ1. The results are shown on the right side of FIG. 9. As shown in the figure, the mutant synthetic ASPD (G33L type) showed cytotoxicity comparable to that of the mature neurons as in the case of the synthesis ASPD (wild-type).

### 7. Dissociation constant between mutant synthetic ASPD and rpASD1 antibody

The synthetic ASPD or mutant synthetic ASPD (G33L) were immobilized, as a ligand, on a sensor chip, an anti-ASPD polyclonal antibody (rpASD1) was allowed to flow as an analyte, and the dissociation constant (K_{D}) was determined by surface plasmon resonance(SPR). As a result, reactivity to the anti-ASPD polyclonal antibody (rpASD1) of the synthetic ASPD was equivalent to that of the mutant synthetic ASPD (G33L).

### 8. Overexpression of 1 and 2 amino acid mutants of hAPP695sw

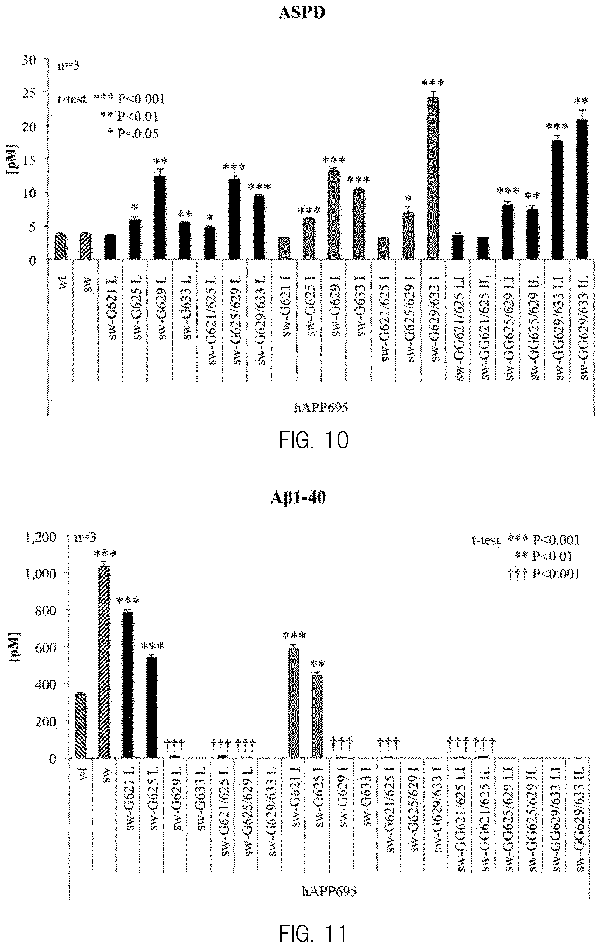

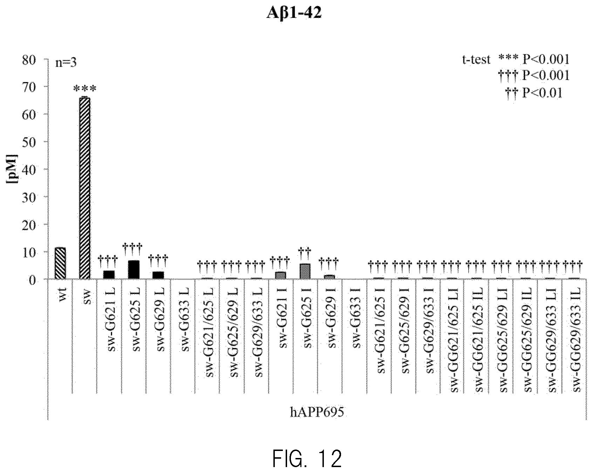

As the amyloid precursor protein (APP) to be expressed, the APPs of Test Examples 15 to 36 shown in Table 3 below and FIG. 1 were used. In the same manner as described in sections 1 and 2 above, the culture medium in which the APP-overexpressing cells had been cultured was collected and the contents of ASPD, Aβ1-40, and Aβ1-42 in the culture medium were confirmed by the ELISA method under the conditions described below. The results are shown in FIGS. 10 to 12.

**[Table 3]**

| Table 3 | APP Expressed (hAAP695) | Mutations in hAAP695 | | Mutations in Aβ |
|---|---|---|---|---|
| Test Example 15 | wild type (WT) | - | | - |
| Test Example 16 | swedish (SW) | KM595/596NL | | - |
| Test Example 17 | sw-G621L | KM595/596NL | G621L | G25L |
| Test Example 18 | sw-G625L | KM595/596NL | G625L | G29L |
| Test Example 19 | sw-G629L | KM595/596NL | G629L | G33L |
| Test Example 20 | sw-G633L | KM595/596NL | G633L | G37L |
| Test Example 21 | sw-G621/625L | KM595/596NL | G621/625L | G25/29L |
| Test Example 22 | sw-G625/629L | KM595/596NL | G625/629L | G29/33L |
| Test Example 23 | sw-G629/633L | KM595/596NL | G629/633L | G33/37L |
| Test Example 24 | sw-G621I | KM595/596NL | G621I | G25I |
| Test Example 25 | sw-G625I | KM595/596NL | G625I | G29I |
| Test Example 26 | sw-G629I | KM595/596NL | G629I | G33I |
| Test Example 27 | sw-G633I | KM595/596NL | G633I | G37I |
| Test Example 28 | sw-G621/625I | KM595/596NL | G621/6251 | G25/29I |
| Test Example 29 | sw-G625/629I | KM595/596NL | G625/629I | G29/33I |
| Test Example 30 | sw-G629/633I | KM595/596NL | G629/633I | G33/37I |
| Test Example 31 | sw-GG621/625LI | KM595/596NL | GG621/625LI | GG25/29LI |
| Test Example 32 | sw-GG621/625IL | KM595/596NL | GG621/625IL | GG25/29IL |
| Test Example 33 | sw-GG625/629LI | KM595/596NL | GG625/629LI | GG29/33LI |
| Test Example 34 | sw-GG625/629IL | KM595/596NL | GG625/629IL | GG29/33IL |
| Test Example 35 | sw-GG629/633LI | KM595/596NL | GG629/633LI | GG33/37LI |
| Test Example 36 | sw-GG629/633IL | KM595/596NL | GG629/633IL | GG33/37IL |

As shown in FIGS. 10 to 12, formation of ASPD was observed in the culture medium in all the cases where APPs of Test Examples 15 to 36 were overexpressed.

There was no significant difference in the contents of ASPD, Aβ1-40, and Aβ1-42 contained in the supernatant between substitution mutation of leucine that substituted 1 amino acid or 2 amino acids and that of isoleucine that substituted 1 amino acid or 2 amino acids. However, the amount of ASPD formed in the case of the APP (Test Example 30) in which both glycines at positions 33 and 37 of Aβ were mutated to isoleucine significantly increased as compared to that in the case of the APP (Test Example 23) in which both glycines at positions 33 and 37 of Aβ were mutated to leucine.

In the case of the APPs (Test Examples 31 to 36) in which two sites were mutated to leucine and isoleucine, the amounts of ASPD formed in the case of the APPs (Test Examples 35 and 36) in which glycines at positions 33 and 37 of Aβ were mutated to leucine and isoleucine increased to the same level as that in the case of the APP (Test Example 30) in which both glycines at positions 33 and 37 of Aβ were mutated to isoleucine.

## Claims

1. A method of producing a cell secreted-type amylospheroids (ASPD)-like structure, the method comprising a step of culturing, in a culture medium, a cell that expresses an amyloid precursor protein (APP) or a part thereof comprising an amyloid beta-protein (Aβ) sequence to obtain a cell secreted-type ASPD-like structure in said culture medium.

2. The production method according to claim 1, further comprising collecting the cell secreted-type ASPD-like structure from the culture medium.

3. The production method according to claim 1 or 2, wherein the cell is a cell in which an expression system of APP or a part thereof comprising an Aβ sequence has been introduced.

4. The production method according to any one of claims 1 to 3, wherein the APP is of a wild-type or is a mutant of the wild-type having one or more amino acid mutations.

5. The production method according to any one of claims 1 to 4, wherein the APP is a mutant APP having an amino acid mutation in glycine in a GXXXG motif of an Aβ amino acid sequence.

6. A cell secreted-type ASPD-like structure, being obtained in a culture medium in which a cell has been cultured, with an expression system that expresses APP or a part thereof comprising an Aβ sequence having been introduced into the cell, the structure being antigenic to an ASPD-specific antibody.

7. A pharmaceutical composition, comprising, as an active ingredient, a cell secreted-type ASPD-like structure according to claim 6.

8. A vaccine, comprising a cell secreted-type ASPD-like structure according to claim 6.

9. A kit, comprising a cell secreted-type ASPD-like structure according to claim 6 and an anti-ASPD antibody.

10. Use of a cell secreted-type ASPD-like structure according to claim 6 as an active vaccine.

11. Use of a cell secreted-type ASPD-like structure according to claim 6 in vaccine production.

12. Use of a cell secreted-type ASPD-like structure according to claim 6 as a reference material in ASPD measurement.

13. A method of preventing, ameliorating, and/or treating a disease caused by ASPD, the method comprising administering, to a subject, a cell secreted-type ASPD-like structure according to claim 6, a pharmaceutical composition according to claim 7, or a vaccine according to claim 8.

14. The method according to claim 13, wherein the disease caused by ASPD is Alzheimer's disease and/or Lewy body dementia.

15. A method of immunizing a subject against ASPD, a disease caused by ASPD, or Alzheimer's disease and/or Lewy body dementia, the method comprising administering, to a subject, a cell secreted-type ASPD-like structure according to claim 6, a pharmaceutical composition according to claim 7, or a vaccine according to claim 8.

16. A method of producing a pharmaceutical composition or a vaccine, the method comprising combining a cell secreted-type ASPD-like structure according to claim 6 with a pharmaceutically acceptable excipient.

17. A method of producing a kit, the method comprising combining a cell secreted-type ASPD-like structure according to claim 6 with an anti-ASPD antibody.

18. A non-human animal, comprising a cell in which an expression system that expresses APP or a part thereof comprising an Aβ sequence has been introduced.

19. A method of screening a substance that affects the formation of ASPD, the method comprising using, as an indicator, the formation efficiency of a structure that is formed in a culture medium in which a cell has been cultured, with an expression system that expresses APP or a part thereof comprising an Aβ sequence having been introduced into the cell, and the structure being antigenic to an ASPD-specific antibody.

20. A method of screening a substance that affects a C-terminal cleavage in Aβ, the method including using, as an indicator, at least one selected from the group consisting of Aβ40, Aβ41, Aβ42, Aβ43, and combinations thereof, which are secreted in a culture medium in which a cell has been cultured, with an expression system that expresses APP or a part thereof comprising an Aβ sequence having been introduced into the cell.

21. A synthetic ASPD-like structure formed by stirring a liquid comprising a mutant Aβ having one or more substitution mutations of a glycine of a GXXXG motif in an amino acid sequence of positions 25 to 37 of Aβ.
